# EUROPEAN PATENT SPECIFICATION

(11) **EP 3 755 229 B1**
(45) Date of publication and mention of the grant of the patent: **09.04.2025**
(21) Application number: 19707347.1
(22) Date of filing: 22.02.2019
(51) Int. Cl.: A61B 8/00, A61B 34/20, G01S 7/52, G01S 15/89, A61B 8/08, A61B 8/14

(54) **INTERVENTIONAL MEDICAL DEVICE TRACKING**
VERFOLGUNG EINER INTERVENTIONELLEN MEDIZINISCHEN VORRICHTUNG
SUIVI DE DISPOSITIF MÉDICAL INTERVENTIONNEL

(30) Priority: 22.02.2018 US 201862633788 P
(43) Date of publication of application: 30.12.2020
(73) Proprietor: Koninklijke Philips N.V., 5656 AG Eindhoven (NL)
(72) Inventor: BHARAT, Shyam, 5656 AE Eindhoven (NL); CHEN, Alvin, 5656 AE Eindhoven (NL); VAIDYA, Kunal, 5656 AE Eindhoven (NL); VIGNON, Francois, Guy, Gerard, Marie, 5656 AE Eindhoven (NL); ERKAMP, Ramon, Quido, 5656 AE Eindhoven (NL); JAIN, Ameet, Kumar, 5656 AE Eindhoven (NL)
(74) Representative: Philips Intellectual Property & Standards
(86) International application number: PCT/EP2019/054399
(87) International publication number: WO 2019/162422

(56) References cited:
- WO-A2-2016/081321
- US-A1- 2005 090 742
- US-A1- 2012 041 311
- US-A1- 2013 197 357

## Description

### BACKGROUND

A transesophageal echocardiography (TEE) ultrasound probe is commonly used in cardiac monitoring and navigation. Currently available multi-plane imaging modes for a TEE ultrasound probe include X-plane and full three-dimensional (3D) volume.

Ultrasound tracking technology estimates the position of a passive ultrasound sensor (e.g., PZT, PVDF, copolymer or other piezoelectric material) in the field of view (FOV) of a diagnostic ultrasound B-mode image by analyzing the signal received by the passive ultrasound sensor as the imaging beams of the ultrasound probe sweep the field of view. Time-of-flight measurements provide the axial/radial distance of the passive ultrasound sensor from the imaging array, while amplitude measurements and knowledge of the beam firing sequence provide the lateral / angular position of the passive ultrasound sensor.

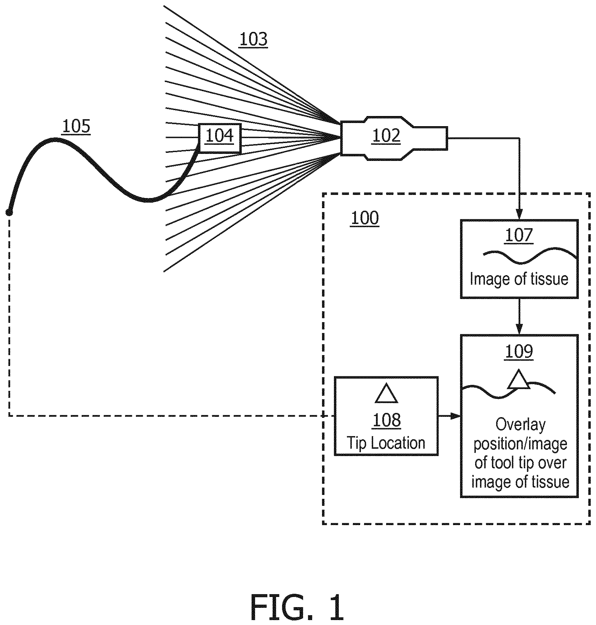

FIG. 1 illustrates a known system for tracking an interventional medical device using a passive ultrasound sensor. In FIG. 1, an ultrasound probe 102 emits an imaging beam 103 that sweeps across a passive ultrasound sensor 104 on a tool tip of an interventional medical device 105. An image of tissue 107 is fed back by the ultrasound probe 102. A location of the passive ultrasound sensor 104 on the tool tip of the interventional medical device 105 is provided as a tip location 108 upon determination by a signal processing algorithm. The tip location 108 is overlaid on the image of tissue 107 as an overlay image 109. The image of tissue 107, the tip location 108, and the overlay image 109 are all displayed on a display 100.

A document US 2012/041311 A1 relates to a system and method of three dimensional acoustic imaging for medical procedure guidance that includes receiving an acoustic signal that is scanned to interrogate a volume of interest; determining a location of a procedural device within the interrogated volume from the acoustic signal; and displaying on a display device a first view of a first plane perpendicular to an orientation of the procedural device. A second view of at least one plane perpendicular to the first plane is also displayed. A third view of a third plane perpendicular to the first plane and to the second plane is also displayed. The first, second, and third views are displayed at the same time

Another document US 2005/090742 A1 discloses ultrasonic diagnostic equipment equipped with an ultrasonic probe that transmits/receives ultrasound to/from an examined body, a probe position sensor that detects the position and the direction of the ultrasonic probe, an image generator that generates image data based upon the output of the ultrasonic probe, a probe position sensor that detects the position and the direction of a puncture probe inserted into the examined body, a display image generator that generates the data of a display image in which the end position of the puncture probe is fixed to a specific position in an image display area according to the position and the direction of the ultrasonic probe and the position and the direction of the puncture probe based upon the image data and a display for displaying the display image in the image display area.

Another document US 2013/197357 A1 discloses a system and method for providing image guidance for placement of one or more medical devices at a target location. The system receives emplacement information of medical devices within a predetermined area. The system calculates a viewing angle in a virtual 3D space of a plurality of virtual medical devices corresponding to the plurality of medical devices. The system also causes a display device to display the plurality of virtual medical devices based at least on the calculated viewing angle.

Another document WO 2016/081321 discloses an ultrasound imaging system that includes an interventional medical device having a first tracking element that generates tip location data based on a locator field. An ultrasound probe has an ultrasound transducer mechanism and a second tracking element. The ultrasound transducer mechanism has an active ultrasound transducer array that generates two-dimensional ultrasound slice data at any of a plurality of discrete imaging locations within a three-dimensional imaging volume. The second tracking element generates probe location data based on the locator field. A processor circuit is configured to execute program instructions to generate an ultrasound image for display, and is configured to generate a positioning signal based on the tip location data and the probe location data to dynamically position the active ultrasound transducer array so that the two-dimensional ultrasound slice data includes the distal tip of the interventional medical device.

### SUMMARY

The invention is defined by the claims. According to one aspect of the present disclosure, a system for tracking an interventional medical device in a patient, is provided. The system comprises: an imaging probe (102, 402A) configured to activate imaging elements to emit imaging beams (103) to generate four or more imaging planes (591, 592, 596, 597, 691, 692, 696, 697) within a field of view including a first imaging plane, a second imaging plane, a third imaging plane perpendicular to the second imaging plane, and a fourth imaging plane perpendicular to the first imaging plane, to simultaneously capture the interventional medical device (105) and anatomy targeted by the interventional medical device (105). The system also comprises a controller (400A, 400B) configured to control the imaging probe (102, 402A) to simultaneously capture both the interventional medical device (105) and the anatomy targeted by the interventional medical device (105), the interventional medical device and targeted anatomy being physically separated in a three-dimensional space, wherein the imaging probe (102, 402A) is controlled to capture at least one of the interventional medical device (105) and the anatomy targeted by the interventional medical device (105) in at least two of the four or more imaging planes (591, 592, 596, 597, 691, 692, 696, 697), and to capture the other of the interventional medical device (105) and the anatomy targeted by the interventional medical device (105) in at least one of the four or more imaging planes (591, 592, 596, 597, 691, 692, 696, 697), wherein the controller (400A, 400B) includes a signal processor that processes image signals that simultaneously capture at least one of the interventional medical device (105) and the anatomy targeted by the interventional medical device (105) in at least two of the four or more imaging planes (591, 592, 596, 597, 691, 692, 696, 697) and the other of the interventional medical device (105) and the anatomy targeted by the interventional medical device (105) in at least one of the four or more imaging planes (591, 592, 596, 597, 691, 692, 696, 697). Controlling the imaging probe (102, 402A) to capture the interventional medical device (105) comprises automatically tracking the interventional medical device with the respective imaging plane(s) based on tracked positions of the interventional medical device (105) determined by analyzing ultrasound signals received by a passive ultrasound sensor (104) disposed on the interventional medical device (105) as the imaging beams of the ultrasound probe (102, 402A) sweep the field of view. The second imaging plane and the third imaging plane are dedicated to capturing the interventional medical device (105), and the first imaging plane and the fourth imaging plane are dedicated to capturing the anatomy targeted by the interventional medical device (105).

According to another aspect of the present disclosure, there is provided a computer program for tracking an interventional medical device (105) in a patient, the program comprising instructions that when executed by the controller of the system of claim 1 cause the controller to carry out the following steps: emitting, by activating imaging elements controlled by the imaging probe (102, 402A), imaging signals to generate four or more imaging planes (591, 592, 596, 597, 691, 692, 696, 697) within the field of view including the first imaging plane, the second imaging plane, the third imaging plane perpendicular to the second imaging plane, and the fourth imaging plane perpendicular to the first imaging plane, to simultaneously capture the interventional medical device (105) and anatomy targeted by the interventional medical device (105), the interventional medical device (105) and targeted anatomy being physically separated in a three-dimensional space. The method also includes: simultaneously capturing the interventional medical device and the anatomy targeted by the interventional medical device, wherein the imaging probe (102, 402A) is controlled to capture at least one of the interventional medical device and the anatomy targeted by the interventional medical device in at least two of the four or more imaging planes (591, 592, 596, 597, 691, 692, 696, 697), and to capture the other of the interventional medical device and the anatomy targeted by the interventional medical device in at least one of the four or more imaging planes (591, 592, 596, 597, 691, 692, 696, 697). Controlling the imaging probe (102, 402A) to capture the interventional medical device comprises automatically tracking the interventional medical device with the respective imaging plane(s) based on tracked positions of the interventional medical device determined by analyzing ultrasound signals received by the passive ultrasound sensor (104) disposed on the interventional medical device as the imaging beams of the ultrasound probe (102, 402A) sweep the field of view. The second imaging plane and the third imaging plane are dedicated to capturing the interventional medical device (105), and the first imaging plane and the fourth imaging plane are dedicated to capturing the anatomy targeted by the interventional medical device (105).

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
FIG. 1 illustrates a known system for interventional medical device tracking using a passive ultrasound sensor, in accordance with a representative embodiment.
FIG. 2 is an illustrative embodiment of a general computer system, on which a method of interventional medical device tracking can be implemented, in accordance with a representative embodiment.
FIG. 3 illustrates a method for interventional medical device tracking.
FIG. 4A illustrates a relationship between a probe and a controller for interventional medical device tracking, in accordance with a representative embodiment.
FIG. 4B illustrates another relationship between a probe and a controller for interventional medical device tracking, in accordance with a representative embodiment.
FIG. 5A illustrates a cross section of a probe for interventional medical device tracking, in accordance with a representative embodiment.
FIG. 5B illustrates a simplified view of imaging planes in the embodiment of FIG. 5A.
FIG. 6A illustrates another cross section of a probe for interventional medical device tracking, in accordance with a representative embodiment.
FIG. 6B illustrates a simplified view of imaging planes in the embodiment of FIG. 6A.
FIG. 7A illustrates another cross section of a probe for interventional medical device tracking, which does not fall under the scope of protection of the present invention.
FIG. 7B illustrates a simplified view of imaging planes of FIG. 7A.
FIG. 8A illustrates another cross section of a probe for interventional medical device tracking, in accordance with a representative embodiment.
FIG. 8B illustrates a simplified view of imaging planes in the embodiment of FIG. 8A.
FIG.9 illustrates views presented on a user interface for interventional medical device tracking, in accordance with a representative embodiment.

### DETAILED DESCRIPTION

In the following detailed description, for purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only, and is not intended to be limiting. The defined terms are in addition to the technical and scientific meanings of the defined terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only, and is not intended to be limiting. As used in the specification and appended claims, the singular forms of terms 'a', 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to", "coupled to", or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In view of the foregoing, the present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments.

As introduced above, use of an X-plane can provide a high frame rate, but only 2 adjustable imaging planes. On the other hand, use of a full three-dimensional (3D) volume can provide control over slicing, but a low frame rate. The present disclosure provides an ability to simultaneously visualize both an interventional medical device and anatomy targeted by the interventional medical device using, for example, the same ultrasound imaging probe by emitting imaging signals in three or more imaging planes. To be clear from the start, the simultaneous emission and capture by the ultrasound imaging probe involves emitting and capturing the interventional medical device and targeted anatomy when the interventional medical device and targeted anatomy are physically separated in a three-dimensional space.

As described for embodiments below, tissue around a device can be visualized with other quantitative navigation metrics, without losing sight of desired anatomy. Device tracking output can be bootstrapped to an imaging plane selection algorithm, via an automatic feedback/control loop that links device location to control of imaging plane selection. An example of an automatic feedback/control loop is a remote control link (RCL), which tracks an identified device through imaging planes as the device is moved. By linking the interventional device tracking output and the imaging plane selection, multiple different embodiments described herein provide varying capabilities. In other words, the interventional device tracking is used as part of a feedback loop to ensure that the ability to track the interventional device continues, so that one or more imaging planes are tied or dedicated to the interventional device. Thus, device tracking can be used to automatically visually follow a device with the imaging planes, in order to continue tracking the interventional device.

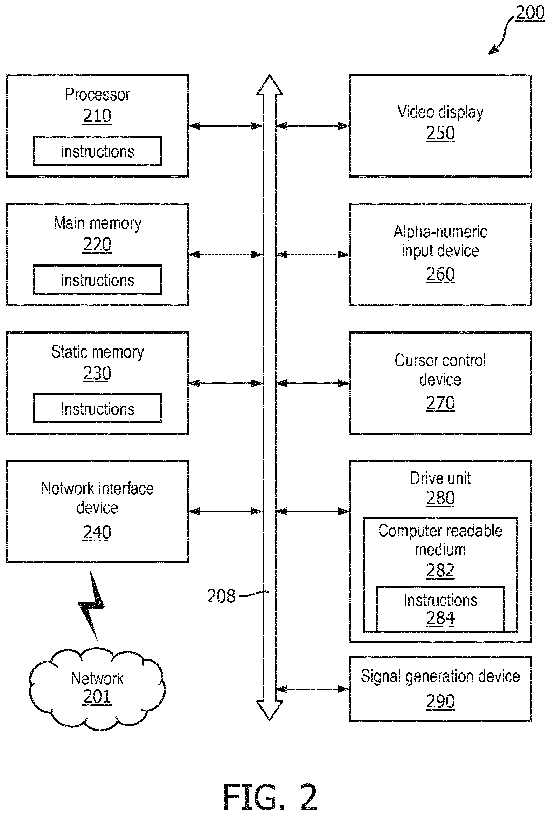

FIG. 2 is an illustrative embodiment of a general computer system, on which a method of interventional medical device tracking can be implemented, in accordance with a representative embodiment. The computer system 200 can include a set of instructions that can be executed to cause the computer system 200 to perform any one or more of the methods or computer based functions disclosed herein. The computer system 200 may operate as a standalone device or may be connected, for example, using a network 201, to other computer systems or peripheral devices.

The computer system 200 can be implemented as or incorporated into various devices, such as a stationary computer, a mobile computer, a personal computer (PC), a laptop computer, a tablet computer, an ultrasound system, an ultrasound probe, or any other machine capable of executing a set of instructions (sequential or otherwise) that specify actions to be taken by that machine. The computer system 200 can be incorporated as or in a device that in turn is in an integrated system that includes additional devices. In an embodiment, the computer system 200 can be implemented using electronic devices that provide voice, video or data communication. Further, while the computer system 200 is illustrated as a single system, the term "system" shall also be taken to include any collection of systems or sub-systems that individually or jointly execute a set, or multiple sets, of instructions to perform one or more computer functions.

As illustrated in FIG. 2, the computer system 200 includes a processor 210. A processor for a computer system 200 is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. A processor is an article of manufacture and/or a machine component. A processor for a computer system 200 is configured to execute software instructions to perform functions as described in the various embodiments herein. A processor for a computer system 200 may be a general-purpose processor or may be part of an application specific integrated circuit (ASIC). A processor for a computer system 200 may also be a microprocessor, a microcomputer, a processor chip, a controller, a microcontroller, a digital signal processor (DSP), a state machine, or a programmable logic device. A processor for a computer system 200 may also be a logical circuit, including a programmable gate array (PGA) such as a field programmable gate array (FPGA), or another type of circuit that includes discrete gate and/or transistor logic. A processor for a computer system 200 may be a central processing unit (CPU), a graphics processing unit (GPU), or both. Additionally, any processor described herein may include multiple processors, parallel processors, or both. Multiple processors may be included in, or coupled to, a single device or multiple devices.

Moreover, the computer system 200 includes a main memory 220 and a static memory 230 that can communicate with each other via a bus 208. Memories described herein are tangible storage mediums that can store data and executable instructions, and are non-transitory during the time instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. A memory described herein is an article of manufacture and/or machine component. Memories described herein are computer-readable mediums from which data and executable instructions can be read by a computer. Memories as described herein may be random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, blu-ray disk, or any other form of storage medium known in the art. Memories may be volatile or non-volatile, secure and/or encrypted, unsecure and/or unencrypted.

As shown, the computer system 200 may further include a video display unit 250, such as a liquid crystal display (LCD), an organic light emitting diode (OLED), a flat panel display, a solid-state display, or a cathode ray tube (CRT). Additionally, the computer system 200 may include an input device 260, such as a keyboard/virtual keyboard or touch-sensitive input screen or speech input with speech recognition, and a cursor control device 270, such as a mouse or touch-sensitive input screen or pad. The computer system 200 can also include a disk drive unit 280, a signal generation device 290, such as a speaker or remote control, and a network interface device 240.

In an embodiment, as depicted in FIG. 2, the disk drive unit 280 may include a computer-readable medium 282 in which one or more sets of instructions 284, e.g. software, can be embedded. Sets of instructions 284 can be read from the computer-readable medium 282. Further, the instructions 284, when executed by a processor, can be used to perform one or more of the methods and processes as described herein. In an embodiment, the instructions 284 may reside completely, or at least partially, within the main memory 220, the static memory 230, and/or within the processor 210 during execution by the computer system 200.

In an alternative embodiment, dedicated hardware implementations, such as application-specific integrated circuits (ASICs), programmable logic arrays and other hardware components, can be constructed to implement one or more of the methods described herein. One or more embodiments described herein may implement functions using two or more specific interconnected hardware modules or devices with related control and data signals that can be communicated between and through the modules. Accordingly, the present disclosure encompasses software, firmware, and hardware implementations. Nothing in the present application should be interpreted as being implemented or implementable solely with software and not hardware such as a tangible non-transitory processor and/or memory.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing can be constructed to implement one or more of the methods or functionality as described herein, and a processor described herein may be used to support a virtual processing environment.

The present disclosure contemplates a computer-readable medium 282 that includes instructions 284 or receives and executes instructions 184 responsive to a propagated signal; so that a device connected to a network 101 can communicate voice, video or data over the network 201. Further, the instructions 284 may be transmitted or received over the network 201 via the network interface device 240.

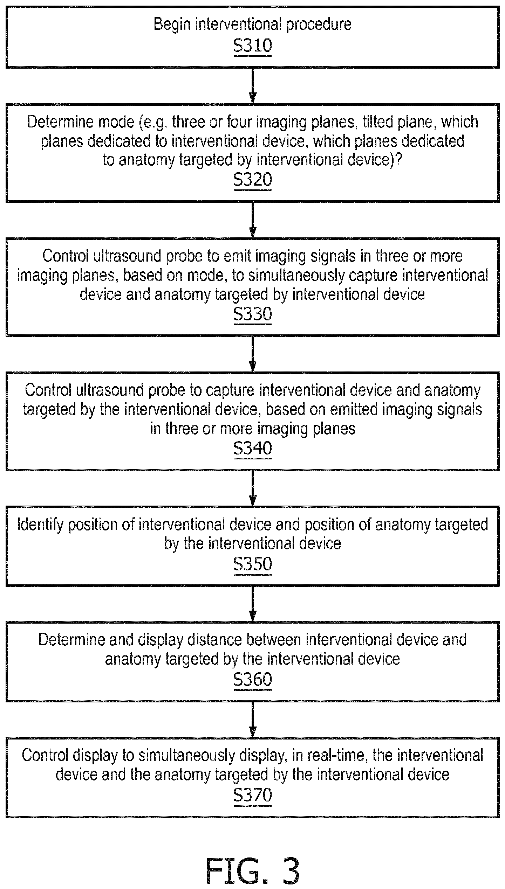

FIG. 3 illustrates a method for interventional medical device tracking.

In FIG. 3, an interventional procedure begins at S310. An interventional procedure is a procedure in which an interventional medical device is partially or fully placed in the body of a patient, such as for exploratory diagnosis or treatment. An interventional medical device may be or may include a wire, an implant, a sensor including a passive ultrasound sensor, or other forms of tangible devices placed into bodies of patients.

At S320, a mode is determined. A mode may consist of a set of one or more selecting settings such as four imaging planes, rotations of planes about an axis, which planes are dedicated to an interventional device, and which planes are dedicated to anatomy targeted by an interventional device. The term "dedicated" as used herein may refer to an assignment of planes to a specific target, which for the purposes of the present disclosure is either an interventional device, or anatomy targeted by the interventional device. The interventional device is targeted by dedicated planes that track the interventional device in two dimensions or three dimensions as the interventional device moves in the body of the patient.

The anatomy targeted by the interventional device may be designated by a user instruction, such as by using a mouse and cursor or a touch screen. The anatomy may be a specific position on the surface of an organ such as a heart or lung, and may be targeted by the interventional device in the sense that the interventional device is moved towards the anatomy targeted by the interventional device. The interventional device may also be designated by a user, but may alternatively be automatically identified and tracked, such as with the use of a sensor made of a specific material that is readily identified in ultrasound.

At S330, an ultrasound probe is controlled to emit imaging signals in three or more imaging planes, based on the mode, to simultaneously capture both the interventional device and the anatomy targeted by the interventional device. In known ultrasound, X-planes use 2 imaging planes, such as 2 perpendicular planes, and capture only one of an interventional device or anatomy targeted by the interventional device. However, at S330, three or more imaging planes are used, and between the three or more imaging planes, the interventional device and the anatomy targeted by the interventional device are both simultaneously captured. For example, each of the three or more imaging planes may specifically intersect with one or both of the interventional device and/or the anatomy targeted by the interventional device.

At S340, the ultrasound probe is controlled to capture both the interventional device and the anatomy targeted by the interventional device, based on the emitted imaging signals in three or more planes. One of the interventional device and the anatomy targeted by the interventional device is captured in at least two of the three or more imaging planes, and the other of the interventional device and the anatomy targeted by the interventional device is simultaneously captured in least one of the three or more imaging planes. In embodiments, both of the interventional device and the anatomy targeted by the interventional device are simultaneously captured in two of the imaging planes, albeit not necessarily the same two imaging planes. In other embodiments, one or the other of the interventional device and the anatomy targeted by the interventional medical device are captured in one and only one of the imagine planes.

At S350, positions of the interventional device and the anatomy targeted by the interventional device are identified, based, for example, on the capture of reflected/returned imaging signals. Alternately, positions of the interventional device can be tracked from signals of a passive ultrasound sensor, or by other methods and mechanisms. Positions may be identified in a predetermined coordinate system, such as in a three-dimensional cartesian coordinate system with dimensions for width (X), height (Y) and depth (Z). A center of the coordinate system may be set at a fixed point in the space (volume) in or around the patient body.

In an embodiment, multiple different medical imaging systems may be registered to one another, so as to reflect commonality in viewpoints. Registration in this manner may involve setting coordinate systems of the different medical systems to reflect a common origin and common directionality dimensions.

At S360, a distance between the interventional device and anatomy targeted by the interventional device is determined and displayed. The distance may be determined in two dimensions, such as width (X)/height (Y), or may be determined in three dimensions such as width (X)/height (Y)/depth (Z).

At S370, a display is controlled to simultaneously display, in real-time, the interventional device and the anatomy targeted by the interventional device. A display may be or may include a screen on a television or on an electronic device such as a monitor. The monitor may be a monitor specifically provided with an ultrasound system, and may have settings specifically appropriate for visualizing imagery captured by the ultrasound system as well as related information such as information related to the captured imagery.

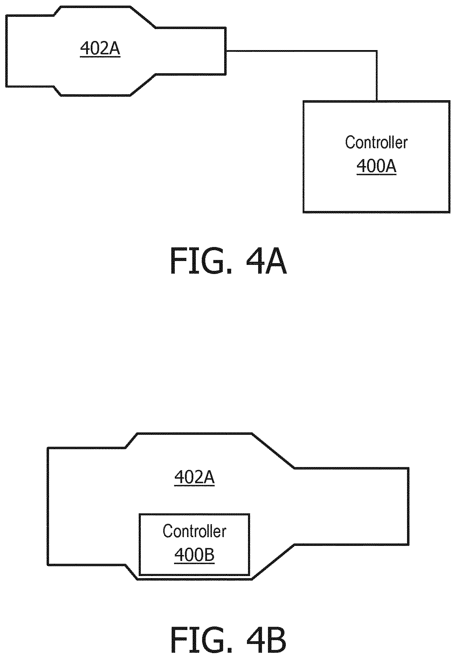

FIG. 4A illustrates a relationship between a probe and a controller for interventional medical device tracking, in accordance with a representative embodiment. In FIG. 4A, a probe 402A is separate from a controller 400A. The probe 402A is an imaging probe, and is controlled to activate imaging elements to emit imaging signals to generate imaging planes that intersect with tissue (e.g., in a patient body). The imaging elements may be transducer elements located on an imaging array. The probe 402A also captures interventional devices and anatomy targeted by the interventional devices in the imaging planes based on the response to the imaging signals (e.g., from the patient body). The probe 402A and controller 400A may communicate wirelessly or by wire. A controller 400A may include a processor 210, a main memory 220 and other elements from the computer system 200 shown in FIG. 2. A controller 400A may execute instructions to perform some or all of the software-based processes described herein, such as some or all of the aspects of the method shown in FIG. 3 herein. Such a controller 400A may be implemented by a computer such as a dedicated ultrasound system that controls a probe 402A and receives and processes imaging data from the probe 402A. Alternatively, a controller 400A may be a distributed subsystem of both the probe 402A and a separate computer that includes the processor 210 and main memory 220 (or other memory).

FIG. 4B illustrates another relationship between a probe and a controller for interventional medical device tracking, in accordance with a representative embodiment. In FIG. 4B, a probe 402A includes a controller 400B. That is, the controller 400B is a component of the probe 402A, and may include elements such as a processor 210 and a main memory 220. The probe 402B is also an imaging probe, and is controlled to activate imaging elements to emit imaging signals to generate imaging planes that intersect with tissue (e.g., in a patient body). The imaging elements may be transducer elements located on an imaging array. The probe 402B also captures interventional devices and anatomy targeted by the interventional devices in the planes based on the response of the tissue (e.g., in the patient body) to the imaging signals. A controller 400B in FIG. 4B may execute instructions to perform some or all of the software-based processes described herein.

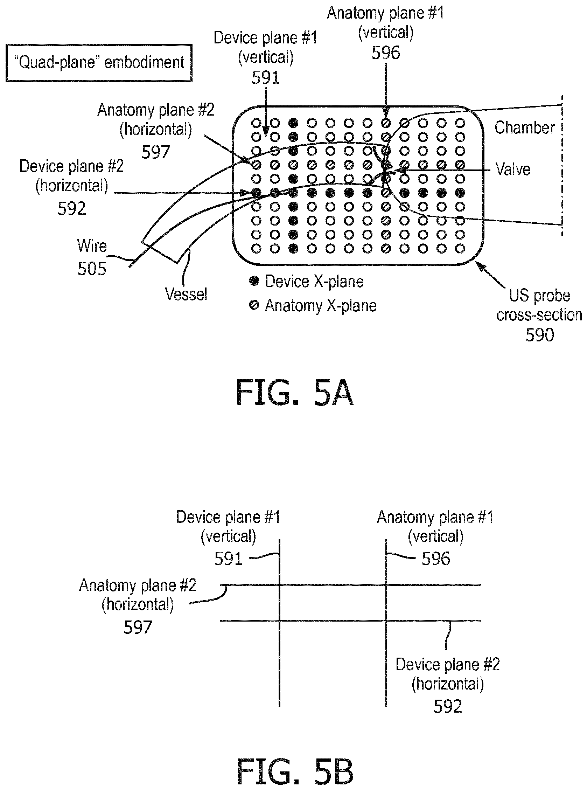

FIG. 5A illustrates a cross section of a probe for interventional medical device tracking, in accordance with a representative embodiment. FIG. 5A shows a "Quad-plane" embodiment in which one X-plane is tied to a device tip and one X-plane is tied to desired anatomy.

FIG. 5A shows the cross-section of the TEE (or other) ultrasound probe on the underlying cardiac anatomy. Active imaging planes are shown by lines of dots. In FIG. 5A, lines of dots in the third column from the left and sixth row from the top are tied to device position, which in turn is obtained from a device tracking method. Lines of dots in the eighth column from the left and fourth row from the top are tied to the desired anatomy, which in turn can be set by the user. Accordingly, in the embodiment of FIG. 5A, two active imaging planes are tied to the interventional device position, and two completely different active imaging planes are tied to the desired anatomy.

Specifically, in FIG. 5A, a wire 505 is overlaid on a vessel and exits the ultrasound probe 590 cross section to the left. A device plane #1 (vertical) 591 and a device plane #2 (horizontal) 592 correspond to the active imaging planes tied to the interventional device position. An anatomy plane #1 (vertical) 596 and an anatomy plane #2 (horizontal) 597 correspond to the active imaging planes tied to the desired anatomy.

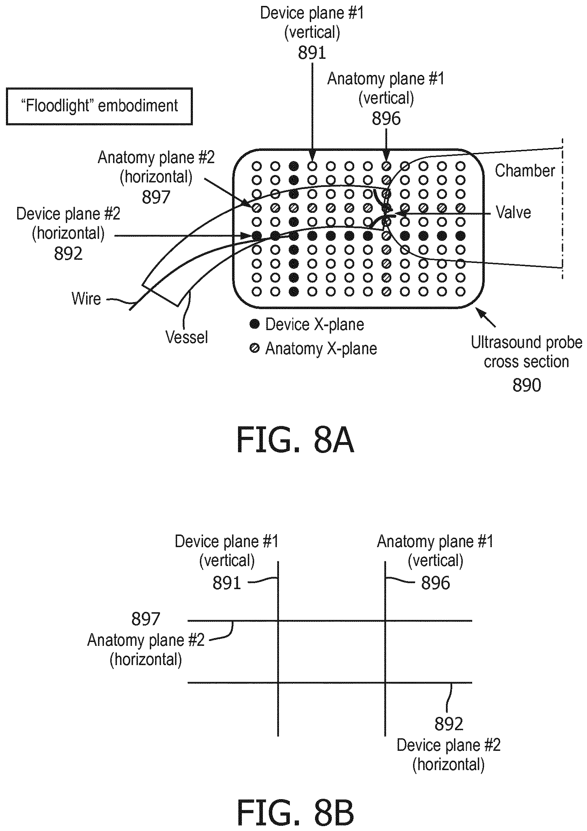

FIG. 5B illustrates a simplified view of imaging planes in the embodiment of FIG. 8A. In FIG. 5B, the device plane #1 (vertical) 591 and the anatomy plane #1 (vertical) 596 are shown as parallel vertical lines. Of course, the device plane #1 (vertical) 591 and the anatomy plane #1 (vertical) 596 do not have to be parallel to each other, or vertical, as these characteristics are used as a referential convenience. Similarly, device plane #2 (horizontal) 592 and anatomy plane #2 (horizontal) 597 are also shown as parallel lines, in this case horizontal lines. The device plane #2 (horizontal) 592 and anatomy plane #2 (horizontal) 597 also do not have to be parallel to each other, or horizontal, as these characteristics are also used only as a referential convenience.

However, the device plane #1 (vertical) 591 and the device plane #2 (horizontal) 592 are shown to be perpendicular, and this characteristic is accurately reflective of how these planes are best used to capture a targeted interventional device or anatomy targeted by an interventional device. Similarly, the anatomy plane #1 (vertical) 596 and anatomy plane #2 (horizontal) 597 are also shown to be perpendicular, and this characteristic is also accurately reflective of how these planes are best used to capture a targeted interventional device or anatomy targeted by an interventional device. Nevertheless, perpendicular planes do not have to be perfectly perpendicular, and may be substantially perpendicular while still working in their intended manner. Examples of substantially perpendicular planes may be intersecting planes with a smaller angle therebetween greater than 67.5 degrees, greater than 75 degrees, or greater than 85 degrees.

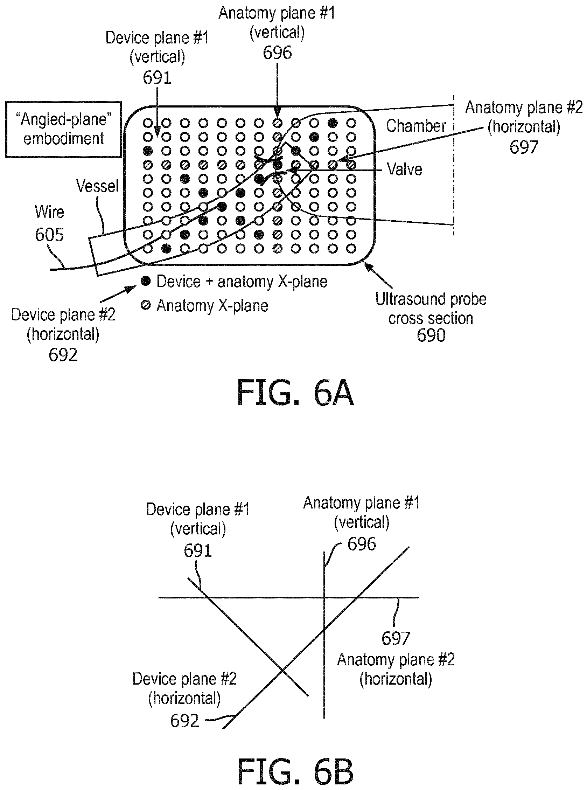

FIG. 6A illustrates another cross section of a probe for interventional medical device tracking, in accordance with a representative embodiment. FIG. 6A shows an "Angled-plane" embodiment in which one X-plane is tied to device and anatomy, and one X-plane is tied to anatomy.

FIG. 6A again shows the cross-section of the TEE (or other) ultrasound probe on the underlying cardiac anatomy. Active imaging planes are shown by lines of dots. In FIG. 6A, lines of dots in the eighth column from the left and fourth row from the top are tied to the desired anatomy, as in the embodiment of FIG. 5A and FIG. 5B. However, the lines of dots tied to the interventional device position are angled by being rotated about an axis to tilt. Accordingly, in the embodiment of FIG. 6A, two active imaging planes are again tied to the interventional device position, but are rotated about an axis to tilt, and two completely different active imaging planes are tied to the desired anatomy.

Specifically, in FIG. 6A, a wire 605 is again overlaid on a vessel and exits the ultrasound probe cross section 690 to the left. A device plane #1 (vertical) 691 and a device plane #2 (horizontal) 692 correspond to the active imaging planes tied to the interventional device position, but both are rotated about an axis to tilt. An anatomy plane #1 (vertical) 696 and an anatomy plane #2 (horizontal) 697 correspond to the active imaging planes tied to the desired anatomy. In the embodiment of FIG. 6A, the "device X-plane" is configured to image the plane containing the interventional device and the desired anatomy.

FIG. 6B illustrates a simplified view of imaging planes in the embodiment of FIG. 6A. In FIG. 6B, the device plane #1 (vertical) 691 and the device plane #2 (horizontal) 692 are rotated about an axis to tilt relative to the embodiment of FIG. 5A and FIG. 5B. However, the device plane #1 (vertical) 691 and the device plane #2 (horizontal) 692 are shown to be perpendicular, and may have the same characteristics as the similar planes in the embodiment of FIG. 5A and FIG. 5B other than their being rotated about an axis to tilt. The anatomy plane #1 (vertical) 696 and anatomy plane #2 (horizontal) 697 are also shown to be perpendicular, and may have the same or similar characteristics to the corresponding planes in the embodiment of FIG. 5A and FIG. 5B.

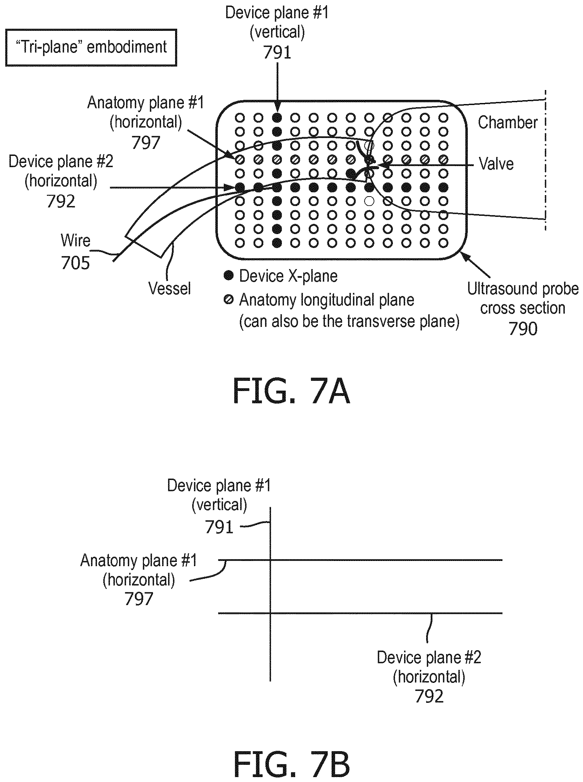

FIG. 7A illustrates another cross section of a probe for interventional medical device tracking. FIG. 7A shows a "Tri-plane" embodiment that is not claimed in which one X-plane is tied to the interventional device tip and one long-axis plane is tied to anatomy.

FIG. 7A again shows the cross-section of the TEE (or other) ultrasound probe on the underlying cardiac anatomy. Active imaging planes are shown by lines of dots. In FIG. 7A, a single line of dots in the fourth row from the top are tied to the desired anatomy. Lines of dots in the third column from the left and sixth row from the top are tied to device position, the same as in the embodiment of FIG. 5A and FIG. 5B described previously.

Accordingly, in the embodiment of FIG. 7A, two active imaging planes are again tied to the interventional device position, but only one completely different active imaging plane is tied to the desired anatomy. In the embodiment of FIG. 7A, the anatomy imaging plane is a single plane, as opposed to a bi-plane, thereby resulting in slightly higher frame rate.

Specifically, in FIG. 7A, a wire 705 is again overlaid on a vessel and exits the ultrasound probe cross section 790 to the left. A device plane #1 (vertical) 791 and a device plane #2 (horizontal) 792 correspond to the active imaging planes tied to the interventional device position. A single anatomy plane #1 (horizontal) 797 corresponds to the active imaging plane tied to the desired anatomy. The anatomy plane #1 (horizontal) 797 is one and the only one imaging plane dedicated to the desired anatomy in the embodiment of FIG. 7A.

In an alternative embodiment, the one anatomy plane #1 (horizontal) 797 can be a short-axis imaging plane rather than a long-axis imaging plane. In still another alternative to the embodiment shown in FIG. 7A, a single X-plane may be assigned to anatomy, and a single plane assigned to the device.

FIG. 7B illustrates a simplified view of imaging planes in the embodiment of FIG. 7A. In the not claimed embodiment of FIG. 7B, the device plane #1 (vertical) 791 is perpendicular or substantially perpendicular to the device plane #2 (horizontal) 792, and the anatomy plane #1 (horizontal) 797 has no corresponding vertical anatomy plane.

FIG. 8A illustrates another cross section of a probe for interventional medical device tracking, in accordance with a representative embodiment.

FIG. 8A shows a "Floodlight"/"look ahead" embodiment in which the transverse plane of the interventional device X-plane is positioned 'x' mm ahead of the tip, to show the "upcoming" anatomy if the interventional device is pushed further.

FIG. 8A shows the cross-section of the TEE (or other) ultrasound probe on the underlying cardiac anatomy. Active imaging planes are shown by lines of dots. In FIG. 8A, lines of dots in the fourth column from the left and sixth row from the top are tied to device position, which in turn is obtained from a device tracking method. Thus, the imaging plane in the fourth column is adjusted based on movement of the interventional device and a current position of the interventional device. In other words, the imaging plane in the fourth column is set based on a trajectory of an intervention in progress, in order to look ahead to show the anatomy that will be encountered when the interventional device is moved further ahead. The current position refers to the position of the interventional device at the time the trajectory is set. Lines of dots in the eighth column from the left and fourth row from the top are tied to the desired anatomy, which in turn can be set by the user. Accordingly, in the embodiment of FIG. 8A, two active imaging planes are tied to the interventional device position, and two completely different active imaging planes are tied to the desired anatomy.

Specifically, in FIG. 8A, a wire 805 is overlaid on a vessel and exits the ultrasound probe 890 cross section to the left. A device plane #1 (vertical) 891 and a device plane #2 (horizontal) 892 correspond to the active imaging planes tied to the interventional device position. An anatomy plane #1 (vertical) 896 and an anatomy plane #2 (horizontal) 897 correspond to the active imaging planes tied to the desired anatomy.

Here, the transverse plane of the interventional device X-plane tied to the interventional device position is adjusted to image the region of tissue "just ahead" of the current device position. The adjusted transverse plane thereby shows which tissue the interventional device will encounter if the interventional device is pushed ahead further in the current direction. Current direction can be determined from the recent history of device positions.

FIG. 8B illustrates a simplified view of imaging planes in the embodiment of FIG. 8A. In FIG. 8B, the various planes are similar to those shown in the embodiment of FIG. 5B. The device plane #1 (vertical) 891 and the device plane #2 (horizontal) 592 are shown to be perpendicular or substantially perpendicular, and the anatomy plane #1 (vertical) 896 and anatomy plane #2 (horizontal) 897 are also shown to be perpendicular or substantially perpendicular. However, as noted above, the device plane #1 (vertical) 891 can be projected based on the position and directionality of the interventional tool, so that the device plane #1 (vertical) 891 can be automatically controlled using feedback from the historical movement and positioning of the interventional tool.

An example of projecting for the embodiments of FIG. 8A and FIG. 8B includes taking the angles of movement over time relative to a vertical axis, a horizontal axis, and a depth axis, particularly if the most recent movement is in a straight line or anything close to a straight line. In this example, the projecting can also take into account speed of movement, such as millimeters per second, in order to identify how far ahead of a current position to target for the anatomy plane #1 (vertical) 896.

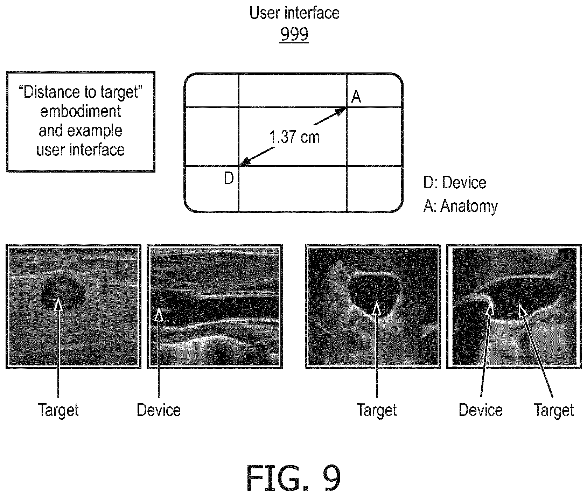

FIG.9 illustrates views presented on a user interface for interventional medical device tracking, in accordance with a representative embodiment.

In FIG. 9, "Distance to target" embodiment: Display distance to anatomical target imaging plane on the interventional device X-plane.

At any time during a procedure, a "distance to target" can be calculated from the current device location and the desired anatomical target, and shown to the user in real-time. This is shown in Figure 9 in conjunction with a sample user interface 999.

Accordingly, interventional medical device tracking enables selective use of different numbers of imaging planes in order to simultaneously capture both an interventional device and anatomy targeted by the interventional device. This provides visualization of tissue around a device and other quantitative navigation metrics, without losing sight of targeted anatomy. Although interventional medical device tracking has been described with reference to several exemplary embodiments, it is understood that the words that have been used are words of description and illustration, rather than words of limitation. Changes may be made within the purview of the appended claims, as presently stated and as amended, without departing from the scope of the appended claims. Although interventional medical device tracking has been described with reference to particular means, materials and embodiments, interventional medical device tracking is not intended to be limited to the particulars disclosed; rather interventional medical device tracking extends to all functionally equivalent structures, methods, and uses such as are within the scope of the appended claims.

## Claims

1. A system for tracking an interventional medical device in a patient, comprising:
an imaging probe (102, 402A) configured to activate imaging elements to emit imaging beams (103) to generate four or more imaging planes (591, 592, 596, 597, 691, 692, 696, 697) within a field of view including a first imaging plane, a second imaging plane, a third imaging plane perpendicular to the second imaging plane, and a fourth imaging plane perpendicular to the first imaging plane, to simultaneously capture the interventional medical device (105) and anatomy targeted by the interventional medical device (105); and
a controller (400A, 400B) configured to control the imaging probe (102, 402A) to simultaneously capture both the interventional medical device (105) and the anatomy targeted by the interventional medical device (105), the interventional medical device and targeted anatomy being physically separated in a three-dimensional space, wherein the imaging probe (102, 402A) is controlled to capture at least one of the interventional medical device (105) and the anatomy targeted by the interventional medical device (105) in at least two of the four or more imaging planes (591, 592, 596, 597, 691, 692, 696, 697), and to capture the other of the interventional medical device (105) and the anatomy targeted by the interventional medical device (105) in at least one of the four or more imaging planes (591, 592, 596, 597, 691, 692, 696, 697), wherein the controller (400A, 400B) includes a signal processor that processes image signals that simultaneously capture at least one of the interventional medical device (105) and the anatomy targeted by the interventional medical device (105) in at least two of the four or more imaging planes (591, 592, 596, 597, 691, 692, 696, 697) and the other of the interventional medical device (105) and the anatomy targeted by the interventional medical device (105) in at least one of the four or more imaging planes (591, 592, 596, 597, 691, 692, 696, 697);
wherein controlling the imaging probe (102, 402A) to capture the interventional medical device (105) comprises automatically tracking the interventional medical device with the respective imaging plane(s) based on tracked positions of the interventional medical device (105) determined by analyzing ultrasound signals received by a passive ultrasound sensor (104) disposed on the interventional medical device (105) as the imaging beams of the ultrasound probe (102, 402A) sweep the field of view; and
wherein the second imaging plane and the third imaging plane are dedicated to capturing the interventional medical device (105), and
the first imaging plane and the fourth imaging plane are dedicated to capturing the anatomy targeted by the interventional medical device (105).

2. The system of claim 1,
wherein the imaging probe (102, 402A) comprises a transesophageal echocardiography (TEE) ultrasound probe.

3. The system of claim 1 further comprising a display, and wherein the controller (400A, 400B) is further configured to control the display to simultaneously display in real-time the interventional medical device (105) and the anatomy targeted by the interventional medical device (105).

4. The system of claim 1, wherein the first imaging plane and the second imaging plane are substantially parallel, and
wherein the third imaging plane and the fourth imaging plane are substantially parallel.

5. The system of claim 1, wherein the second imaging plane and the third imaging plane are configured to capture both the interventional medical device (105) and the anatomy targeted by the interventional medical device (105).

6. The system of claim 1, wherein the controller (400A, 400B) is further configured to control the imaging probe (102, 402A) to rotate the fourth imaging plane and first imaging plane about an axis to tilt the fourth imaging plane and first imaging plane relative to the second imaging plane and the third imaging plane.

7. The system of claim 1, wherein the fourth imaging plane is dedicated to the interventional medical device (105), and
wherein the fourth imaging plane is adjusted to image a region of the anatomy targeted by the interventional device (105) projected based on movement of the interventional medical device (105) and a current position of the interventional medical device (105).

8. computer program for tracking an interventional medical device (105) in a patient, the program comprising instructions that when executed by the controller of the system of claim 1 cause the controller to carry out the following steps:
emitting, by activating imaging elements controlled by the imaging probe (102, 402A), imaging signals to generate four or more imaging planes (591, 592, 596, 597, 691, 692, 696, 697) within a field of view including the first imaging plane, the second imaging plane, the third imaging plane perpendicular to the second imaging plane, and the fourth imaging plane perpendicular to the first imaging plane, to simultaneously capture the interventional medical device (105) and anatomy targeted by the interventional medical device (105), the interventional medical device (105) and targeted anatomy being physically separated in a three-dimensional space; and
simultaneously capturing the interventional medical device and the anatomy targeted by the interventional medical device, wherein the imaging probe (102, 402A) is controlled to capture at least one of the interventional medical device and the anatomy targeted by the interventional medical device in at least two of the four or more imaging planes (591, 592, 596, 597, 691, 692, 696, 697), and to capture the other of the interventional medical device and the anatomy targeted by the interventional medical device in at least one of the four or more imaging planes (591, 592, 596, 597, 691, 692, 696, 697);
wherein controlling the imaging probe (102, 402A) to capture the interventional medical device comprises automatically tracking the interventional medical device with the respective imaging plane(s) based on tracked positions of the interventional medical device determined by analyzing ultrasound signals received by the passive ultrasound sensor (104) disposed on the interventional medical device as the imaging beams of the ultrasound probe (102, 402A) sweep the field of view; and
wherein the second imaging plane and the third imaging plane are dedicated to capturing the interventional medical device (105), and
the first imaging plane and the fourth imaging plane are dedicated to capturing the anatomy targeted by the interventional medical device (105).

9. The method of claim 8, further comprising:
identifying, in a predetermined coordinate system, a position of the interventional medical device and a position of the anatomy targeted by the interventional medical device, and
producing a distance between the position of the interventional medical device and the position of the anatomy targeted by the interventional medical device.

10. The method of claim 8, further comprising:
simultaneously displaying the interventional medical device and the anatomy targeted by the interventional medical device based on the simultaneously capturing the interventional medical device and the anatomy targeted by the interventional medical device.

## Patentansprüche

1. System zum Verfolgen einer interventionellen medizinischen Vorrichtung in einem Patienten, umfassend:
eine Bildgebungssonde (102, 402A), die konfiguriert ist, um Bildgebungselemente zu aktivieren, um Bildgebungsstrahlen (103) auszusenden, um vier oder mehr Bildgebungsebenen (591, 592, 596, 597, 691, 692, 696, 697) innerhalb eines Sichtfelds zu erzeugen, das eine erste Bildgebungsebene, eine zweite Bildgebungsebene, eine dritte Bildgebungsebene senkrecht zur zweiten Bildgebungsebene und eine vierte Bildgebungsebene senkrecht zur ersten Bildgebungsebene beinhaltet, um die interventionelle medizinische Vorrichtung (105) und die von der interventionellen medizinischen Vorrichtung (105) anvisierte Anatomie gleichzeitig aufzunehmen; und
eine Steuereinheit (400A, 400B), die konfiguriert ist, um die Bildgebungssonde (102, 402A) zu steuern, um gleichzeitig beide, die interventionelle medizinische Vorrichtung (105) und die von der interventionellen medizinischen Vorrichtung (105) anvisierte Anatomie aufzunehmen, wobei die interventionelle medizinische Vorrichtung und die anvisierte Anatomie in einem dreidimensionalen Raum physisch getrennt sind, wobei die Bildgebungssonde (102, 402A) gesteuert wird, um mindestens eine von der interventionellen medizinischen Vorrichtung (105) und der von der interventionellen medizinischen Vorrichtung (105) anvisierten Anatomie in mindestens zwei der vier oder mehr Bildgebungsebenen (591, 592, 596, 597, 691, 692, 696, 697) aufzunehmen, und die andere von der interventionellen medizinischen Vorrichtung (105) und der von der interventionellen medizinischen Vorrichtung (105) anvisierten Anatomie in mindestens einer der vier oder mehr Bildgebungsebenen (591, 592, 596, 597, 691, 692, 696, 697) aufzunehmen, wobei die Steuereinheit (400A, 400B) einen Signalprozessor beinhaltet, der Bildsignale verarbeitet, die gleichzeitig mindestens eine von der interventionellen medizinischen Vorrichtung (105) und der von der interventionellen medizinischen Vorrichtung (105) anvisierten Anatomie in mindestens zwei der vier oder mehr Bildgebungsebenen (591, 592, 596, 597, 691, 692, 696, 697) und die andere von der interventionellen medizinischen Vorrichtung (105) und der von der interventionellen medizinischen Vorrichtung (105) anvisierten Anatomie in mindestens einer der vier oder mehr Bildgebungsebenen (591, 592, 596, 597, 691, 692, 696, 697) aufnehmen;
wobei das Steuern der Bildgebungssonde (102, 402A), um die interventionelle medizinische Vorrichtung (105) aufzunehmen, das automatische Verfolgen der interventionellen medizinischen Vorrichtung mit der/den jeweiligen Bildgebungsebene(n) basierend auf verfolgten Positionen der interventionellen medizinischen Vorrichtung (105) umfasst, die durch Analysieren von Ultraschallsignalen bestimmt werden, die von einem passiven Ultraschallsensor (104) empfangen werden, der an der interventionellen medizinischen Vorrichtung (105) angeordnet ist, während die Bildgebungsstrahlen der Ultraschallsonde (102, 402A) das Sichtfeld durchsuchen; und
wobei die zweite Bildgebungsebene und die dritte Bildgebungsebene zum Aufnehmen der interventionellen medizinischen Vorrichtung (105) vorgesehen sind, und
die erste Bildgebungsebene und die vierte Bildgebungsebene zum Aufnehmen der von der interventionellen medizinischen Vorrichtung (105) anvisierten Anatomie vorgesehen sind.

2. System nach Anspruch 1,
wobei die Bildgebungssonde (102, 402A) eine transösophageale Echokardiographie-, (TEE)-Ultraschallsonde umfasst.

3. System nach Anspruch 1, das weiter eine Anzeige umfasst, und wobei die Steuereinheit (400A, 400B) weiter konfiguriert ist, um die Anzeige zu steuern, um die interventionelle medizinische Vorrichtung (105) und die von der interventionellen medizinischen Vorrichtung (105) anvisierte Anatomie gleichzeitig in Echtzeit anzuzeigen.

4. System nach Anspruch 1, wobei die erste Bildgebungsebene und die zweite Bildgebungsebene im Wesentlichen parallel sind, und
wobei die dritte Bildgebungsebene und die vierte Bildgebungsebene im Wesentlichen parallel sind.

5. System nach Anspruch 1, wobei die zweite Bildgebungsebene und die dritte Bildgebungsebene konfiguriert sind, um beide, die interventionelle medizinische Vorrichtung (105) und die von der interventionellen medizinischen Vorrichtung (105) anvisierte Anatomie aufzunehmen.

6. System nach Anspruch 1, wobei die Steuereinheit (400A, 400B) weiter konfiguriert ist, um die Bildgebungssonde (102, 402A) zu steuern, um die vierte Bildgebungsebene und die erste Bildgebungsebene um eine Achse zu drehen, um die vierte Bildgebungsebene und die erste Bildgebungsebene in Bezug zur zweiten Bildgebungsebene und der dritten Bildgebungsebene zu neigen.

7. System nach Anspruch 1, wobei die vierte Bildgebungsebene für die interventionelle medizinische Vorrichtung (105) vorgesehen ist, und
wobei die vierte Bildgebungsebene angepasst ist, um einen Bereich der von der interventionellen Vorrichtung (105) anvisierten Anatomie, die basierend auf Bewegung der interventionellen medizinischen Vorrichtung (105) und einer aktuellen Position der interventionellen medizinischen Vorrichtung (105) projiziert wird, abzubilden.

8. Computerprogramm zum Verfolgen einer interventionellen medizinischen Vorrichtung (105) in einem Patienten, wobei das Programm Anweisungen umfasst, die, wenn sie von der Steuereinheit des Systems nach Anspruch 1 ausgeführt werden, die Steuereinheit veranlassen, die folgenden Schritte durchzuführen:
Aussenden von Bildgebungssignalen durch Aktivieren von Bildgebungselementen, die von der Bildgebungssonde (102, 402A) gesteuert werden, um vier oder mehr Bildgebungsebenen (591, 592, 596, 597, 691, 692, 696, 697) innerhalb eines Sichtfelds zu erzeugen, das die erste Bildgebungsebene, die zweite Bildgebungsebene, die dritte Bildgebungsebene senkrecht zu der zweiten Bildgebungsebene und die vierte Bildgebungsebene senkrecht zu der ersten Bildgebungsebene beinhaltet, um die interventionelle medizinische Vorrichtung (105) und die von der interventionellen medizinischen Vorrichtung (105) anvisierte Anatomie gleichzeitig aufzunehmen, wobei die interventionelle medizinische Vorrichtung (105) und die anvisierte Anatomie physisch in einem dreidimensionalen Raum getrennt sind; und
gleichzeitig Aufnehmen der interventionellen medizinischen Vorrichtung und der von der interventionellen medizinischen Vorrichtung anvisierten Anatomie, wobei die Bildgebungssonde (102, 402A) gesteuert wird, um mindestens eines von der interventionellen medizinischen Vorrichtung und der von der interventionellen medizinischen Vorrichtung anvisierten Anatomie in mindestens zwei der vier oder mehr Bildgebungsebenen (591, 592, 596, 597, 691, 692, 696, 697) aufzunehmen, und um das andere von der interventionellen medizinischen Vorrichtung und der von der interventionellen medizinischen Vorrichtung anvisierten Anatomie in mindestens einer der vier oder mehr Bildgebungsebenen (591, 592, 596, 597, 691, 692, 696, 697) aufzunehmen;
wobei das Steuern der Bildgebungssonde (102, 402A), um die interventionelle medizinische Vorrichtung aufzunehmen, das automatische Verfolgen der interventionellen medizinischen Vorrichtung mit der/den jeweiligen Bildgebungsebene(n) basierend auf verfolgten Positionen der interventionellen medizinischen Vorrichtung umfasst, die durch Analysieren von Ultraschallsignalen bestimmt werden, die vom passiven Ultraschallsensor (104) empfangen werden, der an der interventionellen medizinischen Vorrichtung angeordnet ist, während die Bildgebungsstrahlen der Ultraschallsonde (102, 402A) das Sichtfeld durchsuchen; und
wobei die zweite Bildgebungsebene und die dritte Bildgebungsebene zum Aufnehmen der interventionellen medizinischen Vorrichtung (105) vorgesehen sind, und
die erste Bildgebungsebene und die vierte Bildgebungsebene zum Aufnehmen der von der interventionellen medizinischen Vorrichtung (105) anvisierten Anatomie vorgesehen sind.

9. Verfahren nach Anspruch 8, weiter umfassend:
Identifizieren in einem vorbestimmten Koordinatensystem einer Position der interventionellen medizinischen Vorrichtung und einer Position der von der interventionellen medizinischen Vorrichtung anvisierten Anatomie, und
Herstellen eines Abstands zwischen der Position der interventionellen medizinischen Vorrichtung und der Position der von der interventionellen medizinischen Vorrichtung anvisierten Anatomie.

10. Verfahren nach Anspruch 8, weiter umfassend:
gleichzeitig Anzeigen der interventionellen medizinischen Vorrichtung und der von der interventionellen medizinischen Vorrichtung anvisierten Anatomie basierend auf dem gleichzeitigen Aufnehmen der interventionellen medizinischen Vorrichtung und der von der interventionellen medizinischen Vorrichtung anvisierten Anatomie.

## Revendications

1. Système de suivi d'un dispositif médical interventionnel dans un patient, comprenant :
une sonde d'imagerie (102, 402A) configurée pour activer des éléments d'imagerie pour émettre des faisceaux d'imagerie (103) pour générer quatre, ou plus, plans d'imagerie (591, 592, 596, 597, 691, 692, 696, 697) dans un champ de vision incluant un premier plan d'imagerie, un deuxième plan d'imagerie, un troisième plan d'imagerie perpendiculaire au deuxième plan d'imagerie et un quatrième plan d'imagerie perpendiculaire au premier plan d'imagerie, pour capturer simultanément le dispositif médical interventionnel (105) et une anatomie ciblée par le dispositif médical interventionnel (105) ; et
un dispositif de commande (400A, 400B) configuré pour commander la sonde d'imagerie (102, 402A) pour capturer simultanément à la fois le dispositif médical interventionnel (105) et l'anatomie ciblée par le dispositif médical interventionnel (105), le dispositif médical interventionnel et l'anatomie ciblée étant séparés physiquement dans un espace tridimensionnel, dans lequel la sonde d'imagerie (102, 402A) est commandée pour capturer au moins l'un du dispositif médical interventionnel (105) et de l'anatomie ciblée par le dispositif médical interventionnel (105) dans au moins deux des quatre, ou plus, plans d'imagerie (591, 592, 596, 597, 691, 692, 696, 697), et pour capturer l'autre du dispositif médical interventionnel (105) et de l'anatomie ciblée par le dispositif médical interventionnel (105) dans au moins l'un des quatre, ou plus, plans d'imagerie (591, 592, 596, 597, 691, 692, 696, 697), dans lequel le dispositif de commande (400A, 400B) inclut un processeur de signal qui traite des signaux d'image qui capturent simultanément au moins l'un du dispositif médical interventionnel (105) et de l'anatomie ciblée par le dispositif médical interventionnel (105) dans au moins deux des quatre, ou plus, plans d'imagerie (591, 592, 596, 597, 691, 692, 696, 697) et l'autre du dispositif médical interventionnel (105) et de l'anatomie ciblée par le dispositif médical interventionnel (105) dans au moins l'un des quatre, ou plus, plans d'imagerie (591, 592, 596, 597, 691, 692, 696, 697) ;
dans lequel la commande de la sonde d'imagerie (102, 402A) pour capturer le dispositif médical interventionnel (105) comprend un suivi automatique du dispositif médical interventionnel avec le ou les plans d'imagerie respectifs sur la base de positions suivies du dispositif médical interventionnel (105) déterminées en analysant des signaux ultrasonores reçus par un capteur ultrasonore passif (104) disposé sur le dispositif médical interventionnel (105) lorsque les faisceaux d'imagerie de la sonde ultrasonore (102, 402A) balayent le champ de vision ; et
dans lequel le deuxième plan d'imagerie et le troisième plan d'imagerie sont dédiés à la capture du dispositif médical interventionnel (105), et
le premier plan d'imagerie et le quatrième plan d'imagerie sont dédiés à la capture de l'anatomie ciblée par le dispositif médical interventionnel (105).

2. Système selon la revendication 1,
dans lequel la sonde d'imagerie (102, 402A) comprend une sonde ultrasonore d'échocardiographie transœsophagienne (TEE).

3. Système selon la revendication 1 comprenant en outre un affichage et dans lequel le dispositif de commande (400A, 400B) est en outre configuré pour commander l'affichage pour afficher simultanément en temps réel le dispositif médical interventionnel (105) et l'anatomie ciblée par le dispositif médical interventionnel (105).

4. Système selon la revendication 1, dans lequel le premier plan d'imagerie et le deuxième plan d'imagerie sont sensiblement parallèles, et
dans lequel le troisième plan d'imagerie et le quatrième plan d'imagerie sont sensiblement parallèles.

5. Système selon la revendication 1, dans lequel le deuxième plan d'imagerie et le troisième plan d'imagerie sont configurés pour capturer à la fois le dispositif médical interventionnel (105) et l'anatomie ciblée par le dispositif médical interventionnel (105).

6. Système selon la revendication 1, dans lequel le dispositif de commande (400A, 400B) est en outre configuré pour commander la sonde d'imagerie (102, 402A) pour faire tourner le quatrième plan d'imagerie et le premier plan d'imagerie autour d'un axe pour incliner le quatrième plan d'imagerie et le premier plan d'imagerie par rapport au deuxième plan d'imagerie et au troisième plan d'imagerie.

7. Système selon la revendication 1, dans lequel le quatrième plan d'imagerie est dédié au dispositif médical interventionnel (105), et
dans lequel le quatrième plan d'imagerie est ajusté pour imager une région de l'anatomie ciblée par le dispositif interventionnel (105) projetée sur la base d'un mouvement du dispositif médical interventionnel (105) et d'une position actuelle du dispositif médical interventionnel (105).

8. Programme informatique pour suivre un dispositif médical interventionnel (105) dans un patient, le programme comprenant des instructions qui, lorsqu'elles sont exécutées par le dispositif de commande du système selon la revendication 1, amènent le dispositif de commande à effectuer les étapes suivantes consistant à :
émettre, en activant des éléments d'imagerie commandés par la sonde d'imagerie (102, 402A), des signaux d'imagerie pour générer quatre, ou plus, plans d'imagerie (591, 592, 596, 597, 691, 692, 696, 697) dans un champ de vision incluant le premier plan d'imagerie, le deuxième plan d'imagerie, le troisième plan d'imagerie perpendiculaire au deuxième plan d'imagerie et le quatrième plan d'imagerie perpendiculaire au premier plan d'imagerie, pour capturer simultanément le dispositif médical interventionnel (105) et l'anatomie ciblée par le dispositif médical interventionnel (105), le dispositif médical interventionnel (105) et l'anatomie ciblée étant séparés physiquement dans un espace tridimensionnel ; et
capturer simultanément le dispositif médical interventionnel et l'anatomie ciblée par le dispositif médical interventionnel, dans lequel la sonde d'imagerie (102, 402A) est commandée pour capturer au moins l'un du dispositif médical interventionnel et de l'anatomie ciblée par le dispositif médical interventionnel dans au moins deux des quatre, ou plus, plans d'imagerie (591, 592, 596, 597, 691, 692, 696, 697) et pour capturer l'autre du dispositif médical interventionnel et de l'anatomie ciblée par le dispositif médical interventionnel dans au moins l'un des quatre, ou plus, plans d'imagerie (591, 592, 596, 597, 691, 692, 696, 697) ;
dans lequel la commande de la sonde d'imagerie (102, 402A) pour capturer le dispositif médical interventionnel comprend un suivi automatique du dispositif médical interventionnel avec le ou les plans d'imagerie respectifs sur la base de positions suivies du dispositif médical interventionnel déterminées en analysant des signaux ultrasonores reçus par le capteur ultrasonore passif (104) disposé sur le dispositif médical interventionnel lorsque les faisceaux d'imagerie de la sonde ultrasonore (102, 402A) balayent le champ de vision ; et
dans lequel le deuxième plan d'imagerie et le troisième plan d'imagerie sont dédiés à la capture du dispositif médical interventionnel (105), et
le premier plan d'imagerie et le quatrième plan d'imagerie sont dédiés à la capture de l'anatomie ciblée par le dispositif médical interventionnel (105).

9. Procédé selon la revendication 8, comprenant en outre :
l'identification, dans un système de coordonnées prédéterminé, d'une position du dispositif médical interventionnel et d'une position de l'anatomie ciblée par le dispositif médical interventionnel, et
la production d'une distance entre la position du dispositif médical interventionnel et la position de l'anatomie ciblée par le dispositif médical interventionnel.

10. Procédé selon la revendication 8, comprenant en outre :
l'affichage simultané du dispositif médical interventionnel et de l'anatomie ciblée par le dispositif médical interventionnel sur la base de la capture simultanée du dispositif médical interventionnel et de l'anatomie ciblée par le dispositif médical interventionnel.
